# EUROPEAN PATENT APPLICATION

(11) **EP 0 740 939 A1**
(43) Date of publication of application: **06.11.1996**
(21) Application number: 95106582.0
(22) Date of filing: 02.05.1995
(51) Int. Cl.: A61K 35/14

(54) **Compositions for treatment and prophylaxis of atherosclerosis**

(71) Applicant: DOINA INTERNATIONAL LTD., Road Town Tortola (VG)
(72) Inventor: Doina, Filip, Dr., Bucharest (RO)
(74) Representative: Benedum, Ulrich Max, Dr.

(57) **Abstract**

A pharmaceutical composition for treatment and prophylaxis of atherosclerosis comprising antigenic and immunogenic heavy microemulsion lipid particles. These particles are obtained by, firstly, *isolating* LDL, VLDL, β-VLDL or IDL from the blood of a mammal or human subject; and, secondly, by *reorganizing* the structure of the lipid particles or droplets through an *in-vitro* fusion of said lipoprotein material to obtain heavy microemulsion lipid particles, having a floatation density of 1.17 to 1.24 mg/ml and being ptentially antigenic and immunogenic in mammals or humans.

## Description

### Field of the Invention

The invention relates to compositions comprising heavy microemulsion particles of atherogenic lipid material and to pharmaceutical preparations for treatment and prophylaxis of *atherosclerosis* in mammals and humans. The invention also relates to a method for obtaining a pharmaceutical compositions against atherosclerosis.

### Background of the Invention

*Atherosclerosis* is a widespread human disease manifested by degenerative changes of the structure of the blood vessels. The degenerations are often found at areas next to vessel branching points which are known to be prone to lesions or in regions with a turbulent blood flow due to the morphology of the circulatory system. The degenerative changes are caused by an accumulation of extracelluar lipids, lipid-loaden macrophages (foam cells) and extracelluar matrices and/or by a proliferation of vascular smooth muscle cells. They have all in common that they reduce the vascular lumen and may finally, with all known pathological consequences, obstruct the vascular flow.

A population especially at risk are familial hypercholesterolemics who suffer from a defective apolipoprotein B receptor and impaired clearance of low density lipoprotein (LDL), the main cholesterol carrier, from the blood. Thus, the LDL is transported by a non-receptor mediated mechanism into the vessel intima which leads to more frequent and larger atherogenic lesions.

In type IIb hyperlipoproteinemias the higher atherogenic risk has been associated with lipoproteins having a pre-β electrophoretic migration pattern, the so-called very low density lipoproteins (VLDL) (Gianturco, S.H., et al. *J. Clin. Invest.*, 1982, 70, 168). Floating β lipoproteins (β-VLDL) have also a high atherogenic potential in type III hyperlipoproteinemias (Bates, S.R., *J. Lipid. Res.*, 1987, 28, 787). Moreover, lipoproteins with the characteristics of VLDLs have been isolated from atherosclerotic human aortas (Rapp, J.H., et al, *Arterioscl. Thromb.*,1994, 14, 1767). Quantitative studies as to lipoprotein transfer into the vessel intima have shown in humans that another important atherogenic lipoprotein is the intermediate density lipoprotein (IDL) which is also called VLDL remnant (Shaikh, M., et al, *Arterioscl. Thromb*., 1991, 11, 569).

The present treatments for atherosclerosis try (i) to lower the level of circulating cholesterol and, implicitly, the amounts of circulating LDL, VLDL and IDL, which is achieved by lipid-poor diets and lipid-lowering drugs, and (ii) to counteract modifications which are thought to make the lipoprotein material more atherogenic. The latter is achieved by the administration of antioxidants of the *probucol* and *carotene* type. The extreme cases of the genetic hyperlipidemias and cholesterolemics are treated by the two above methods and by LDL apheresis (Scholzek, P. et al., *Clinical Investigator*, 1992, 70, 99). All these treatment cause great physical and psychological stress to the patients while they can only partially alleviate the symptoms.

Thus, it is an object of the present invention to provide a pharmaceutical composition for effective treatment and prophylaxis of atherosclerosis in mammals and humans and a method for obtaining a composition for immunization against atherosclerosis.

### Detailed Description of the Invention

This aim is achieved by a pharmaceutical composition comprising heavy microemulsion lipid particles obtainable by (i) *isolation* of low density lipoproteins (LDL) and/or very low density lipoproteins (VLDL) and/or intermediate density lipoproteins (IDL) and (ii) *in-vitro*-fusion of said lipoprotein material to obtain heavy microemulsion lipid particles having a floatation density of 1.17 to 1.24 mg/ml.

In a preferred embodiment of the invention, the isolated lipoprotein material is chemically similar to potentially atherogenic lipoprotein material in the blood of the individual to be treated.

Another preferred embodiment of the invention comprises the *in vitro* fusion of VLDL particles with themselves and the fusion of VLDL with LDL particles. As shown below, the VLDL and LDL particles can be fused *in vitro* by fusogenic peptides or osmotic stress. As the IDL particles are chemically intermediate between VLDL and LDL and as IDL particles have dimensions and a radius of curvature close to LDL particles it is obvious for a person skilled in the art that also IDL particles can be fused *in vitro*. Thus, it can be expected that the fusion conditions as shown below are also effective for IDL.

The pharmaceutical preparations for treatment and prophylaxis of atherosclerosis are based on the hypothesis of an active immunization against reorganized lipid material which is immunologically similar to the material appearing in lesion-prone areas. In other words, to illicit the immune system to attack the atherogenic lipid material and to have it removed by phagocytosis.

Moreover, the mere existence of an immune-based lipid clearance system effects irrespective the level of circulating cholesterol a reversion of the lesion-prone areas to their normal state. And the reversion takes only approximately 1/100th of the time for the development of the lesion. Thereafter, a balanced situation is maintained by the immune system.

Thus, the invention is directed to a pharmaceutical preparation comprising as an active ingredient an antigenic reorganized lipid material and a conventional immunization adjuvant.

The invention also relates to a method for preparing a pharmaceutical composition wherein the reorganized antigenic lipid material is obtained by adding *in vitro* an amphipathic material to patient type lipid material so that the lipids fuse and give heavy microemulsion particles. A preferred aspect in this connection is the fusion of VLDL particles with themselves and the fusion of VLDL with LDL particles. In addition, a fusion of IDL particles with themselves and with other lipid particles is contemplated. The antigenic emulsion particles are then combined with a suitable pharmaceutical carrier or adjuvant.

The treatment and prophylaxis is basically a conventional immunization scheme comprising the repeated administration of the above-mentioned pharmaceutical composition, preferably in two-weekly intervals.

Although the invention is not be bound to any theory it is believed that the lipid material in atherogenic lesions is formed by a lipid reorganization in the vascular intima. This fusion or reorganization process produces heavy microemulsion droplets which have multiple copies of apolipoprotein B in their surface. This may also effect that some of the apolipoprotein B molecules expose new antigenic determinants so that the heavy microemulsion particle becomes antigenic and immunogenic.

The fusion of the LDL or other lipoprotein particles can be obtained *in vitro* by conditions which essential exclude the possibility that the native LDL or lipoprotein is modified in terms of oxidation state. Basically, the same ionic conditions are reproduced *in vitro* which most probably excite the LDL in an intima to undergo an atherogenic transformation.

Normal animals such as hamsters have been immunized with an experimental material which only contained a reorganized LDL material in physiological saline. With this material it was possible to obtain specific B and T lymphocyte clones whose antibodies reacted with the *in-vivo* reorganized LDL material, immediately after it was formed. In other words, this was an LDL material of the type which is found in lesion-prone areas of animals on an atherogenic diet. The Syrian golden hamster was used in these experiments because of the great similarities between its lipoprotein metabolism and the human lipid metabolism (Nistor, A., et al., Atherosclerosis, 1987 68, 159). Moreover, it was shown that this animal develops obstructive coronary lesions after long hypercholesterolemic diets (Spady, D.K., et al., *Proc. Natl. Acad. Sci.*, USA, 1983, 80, 3499).

As the reorganized forms of LDL, VLDL, and IDL exist only in lesional areas, only these areas will be the target for the immune-competent lymphocyte clones which induce a reversion of the affected lesion-prone areas to the normal state. As the reversion will also be maintain, this treatment can be applied both prophylactically and therapeutically. The normal LDL metabolism will not be affected since the circulating form of the lipoprotein is the unmodified form.

The essence of the invention is the development of a fusion procedure leading *in vitro* to a lipid- or LDL-based material which is almost identical to the *in vivo* material but which is antigenic and immunogenic. Thus, the *in vitro* material representing a non-self structure which has the capacity to select specific lymphocyte clones which produce antibodies against the extracelluar *in vitro* material and also the atherogenic *lesional* lipid material.

*In-vitro* fusion is preferably done through amphipathic molecules having characteristically hydrophilic and hydrophobic properties. The peptides *bacitracin* and *vasopressin* are such amphipathic molecules and able to trigger fusion of lipid material. The peptides have common structural features which are probably involved in the destabilization of the exterior phospholipid monolayer of the LDL or VLDL particles: the first step of the fusion reaction.

It is also contemplated to use the fusogenic properties of *somatostatin* and of *endothelins*. *Somatostatin*, a neuropeptide, is a tetradecapeptide (MW 1640) with a 12 amino acid ring, which is disulphide bonded, and two neutral amino acids at its carboxy-terminal end. It is thought that the lack of a charge at the carboxy end is compensated by the larger cyclic structure. The *endothelins* are vasoconstrictor peptides of 21 amino acids (MW 2492). They have a globular amino terminus which is stabilized by two disulphide bridges and an amphipathic α-helical carboxy terminus of 6 amino acids (Janes, R.W., et al., *Nature Structural Biology*, 1994, 1, 311). *Endothelins* have been shown to be a marker for arterial diseases, being increased in advanced atheroscleroses (Lerman, A., et al., *New Engl. J. Med.*, 1991, 325, 997). Thus, they are also likely to be suitable agents for fusion of LDL, VLDL and other lipid particles. Moreover, it is obvious to use the common physio-chemical and structural characteristics for finding other potentially fusogenic, amphipathic substances and molecules, including synthetic ones.

Further aspects and advantages of the present invention will become obvious from the attached claims, the description, the representative examples, and the attached figures, wherein:-
**Fig. 1** shows the kinetics of a fusion process of hamster LDL. The fusion was induced by *bacitracin*. The process was monitored by measuring the turbidity and the adsorption of the solution at 400nm (black circles). Each value represents the mean of three aliquots. As shown by the kinetics, fusion was essentially completed in 7 minutes. The equilibrium was reached after 9 minutes.
   For human LDL the kinetics are somewhat slower, the fusion being complete in 15 minutes; the equilibrium being attained after 20 min.
   The same types of measurements were done for the initial and final absorption values in stress-induced fusion experiments (triangle).
**Fig. 2A** shows an electron micrograph (x 95,400) of LDL particles, negative staining. The particles have a similar size of about 20 nm.
**Fig. 2B** shows a photograph of a density gradient in which normal LDL particles, having a flotation density of about 1.06 g/ml, were concentrated in a single band.
**Fig. 2C** shows an electron micrograph (x 181,400), negative staining, of a fused LDL preparation wherein the microemulsion droplets have a size between 30 and 100 nm.
**Fig. 2D** shows a photograph of a vertical density gradient centrifugation wherein the microemulsion droplets have banded, due to multiple copies of apolipoprotein B on their surface, at higher floatation densities of 1.20 and 1.24 mg/ml.
**Fig. 2E** shows an electron micrograph (x 95,400) of microemulsion droplets which have basically the same organizational pattern: a core of neutral lipids (here shown by a cytochemical reaction with tannic acid-paraphenylene diamine) which is stabilized by a phospholipid monolayer.
**Fig. 3A** shows an electron micrograph (x 95,400) of lipid material in immunized animals, post-immunization, starting with a first week of hypercholesterolemic diet. The lipid material of the microemulsion is stabilized in comparison to that of control hypercholesterolemic animals which show the same type of material but a more dynamic reorganization of the lipid components.
**Fig. 3B** shows plasma cells by ultrastructural criteria (x 29,400). The cells are surrounded by extracelluar accumulations of lipid material and have a distended rough endoplasmic reticulum indicative for antibody synthesis (insert x 59, 600).
**Fig. 4.** At 14 days of hypercholesterolemic diet. The previous fibrolipidic-like lesions are transformed into fatty streaks. No more extracelluar lipid material in microemulsion form is found extracellularly. This demonstrates the reversion of the previous fibrolipidic-like lesion to a more benign form of atherogenic lesions, namely the fatty streaks.
**Fig. 5A.** At 30 days of hypercholesterolemic diet. The foam cells are massively regressing from the tissue (x 8,300).
**Fig. 5B.** The foam cells have a specialized interdigitized structure, assuring a cooperative action in the region of the endothelial cell junctions (x 29,400).
**Fig. 6A.** A hyperimmunized animal, post-immunization, at 30 days of diet. The lesional area is already re-equilibrated between microemulsion droplet formation and the disposal by foam cell regression resulting in a normal view of the lesion-prone area (x 54,600).
**Fig. 6B**. A view of the same area in normal control animals. Some microemulsion droplets can be seen immediately under the endothelial cells. Their presence reflects the very high propensity that this area will develop an atherogenic lesion (x 37,800).
**Fig. 6C.** As a control, the region in immunized animals not receiving an atherogenic diet (x 54,600).

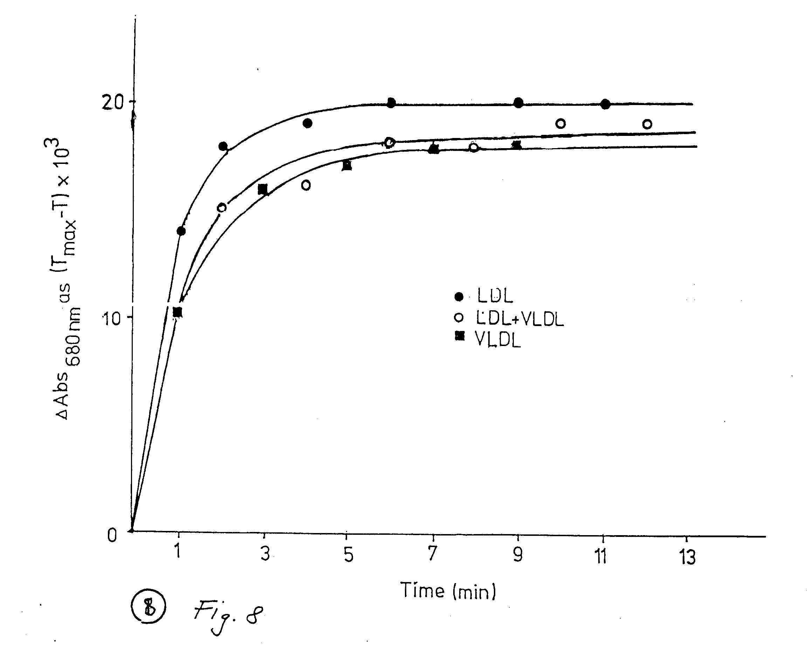

**Fig. 7A.** A view of lesions in an animal which received an atherogenic diet and, after 3 months, a course of injections of modified lipoproteins for 3-4 weeks. The animals were sacrificed after a total hypercholesterolemic diet of 17 weeks, about 10 days after completion of the immunization scheme. Lesion regression is evident though the sequelae of the initial diet period are present, especially in the form of cholesteryl ester droplets (x 54,600). However, even this type of material was removed through a characteristic type of phagocytosis by the monocytes and macrophages in this region (insert x 18,600).
**Fig. 7B.** A view of the atherogenic lesions of controls at approximately the same diet time (x 3,600).

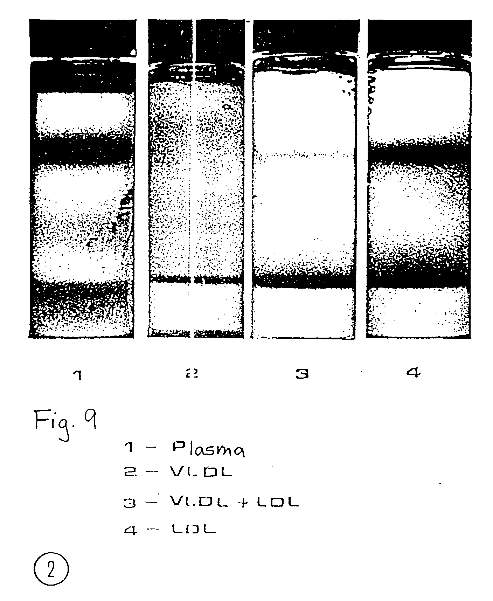

**Fig. 8** shows fusion kinetics of human LDL (0.2 mg/ml), a heterogeneous preparation of equal human LDL and VLDL (0.1 mg/ml), and human VLDL (0.2 mg/ml). The amphipathic fusogen *bacitracin* was added at 0.88 mM final concentration. In all preparations a plateau was reached after 9 to 11 minutes.
**Fig. 9** shows a potassium bromide density gradient centrifugation of heavy microemulsions which were prepared by a fusion of LDL, a heterogeneous LDL and VLDL preparation, and of VLDL. A 1.24 g/ml band was obtained in all three preparations fused by *bacitracin*, 0.88 mM final concentration. The control was a plasma preparation.
**Fig. 10** shows fusion kinetics of human LDL 0.15 mg/ml which was fused *in vitro* by *bacitracin*, *endothelin*-1, *vasopressin*, and *somatostatin*, 60 µm final concentration. The fusion curves show strong fusogenic strength for *bacitracin* and *endothelin*-1 and medium fusogenic strength for *vasopressin* and *somatostatin*. In all cases a plateau was reached after 9 to 11 min.
**Fig. 11** shows that the obtained heavy microemulsion particles had different densities in a KCl density gradient ultracentrifugation. The densities of the bands directly correlate to the fusogenic strength of the tested fusogen.

### Materials and Methods

The *in vitro* work was done with animals and human material. The immunization work was done in animal.

### LDL Isolation and Characterization

Standard methods were applied for the LDL isolation and characterization. From the experimental animals the blood was obtained by cardiac puncture. From human subjects (blood donors) the blood was obtained by phlebotomy in standard conditions after their informed consent. Plasma was immediately obtained by low speed centrifugation. The LDL fraction was obtained from fresh plasma by single vertical density gradient ultracentrifugation (Chung, B.H., et al., *J. Lipid Res.*, 1980, 21, 284). The LDL purity was checked by immunochemical methods using affinity-purified anti holo-LDL polyclonal antibodies. The native state of the LDL was checked by high performance liquid chromatography (HPLC) and quantitative analysis of its malondialdehyde (MDA) content. The levels found characterized the isolated lipoprotein as native (Berliner, J.A., *J. Clin.Invest.*, 1980, 85, 1260). The integrity of the LDL apolipoprotein B, characterizing the native state, was checked by sodium dodecylsulfate polyacrylamid gel electrophoresis (SDS-PAGE).

### In-vitro Fusion

The LDL was transferred into half-diluted phosphate-buffered saline (PBS) containing 72 mM Na⁺, 2 mM K⁺, 0.3 nM Mg²⁺, 0.45 Mm Ca²⁺, by gel filtration on a 2 ml Sephadex G-25 column. The concentration of Ca²⁺ was then raised to 1,6 mM. These ionic concentrations of the fusion buffer (FB) were found essential for the fusion procedure.

It was found that LDL fusion can be obtained *in vitro* by two methods based on entirely different physio-chemical phenomena.

The first method uses is based on the use of amphipathic molecule such as the antibiotic *bacitracin* to induce in-vitro fusion of the lipoproteins. The antibiotic was then removed from the fused preparation by dialysis as it has a small molecular weight. After dialysis the full removal of the antibiotic from the preparation was proven by a specific HPLC method quantitative for *bacitracin*.

The same fusion effect was also found for the neurohormone *vasopressin* which has similar structural features as the antibiotic.

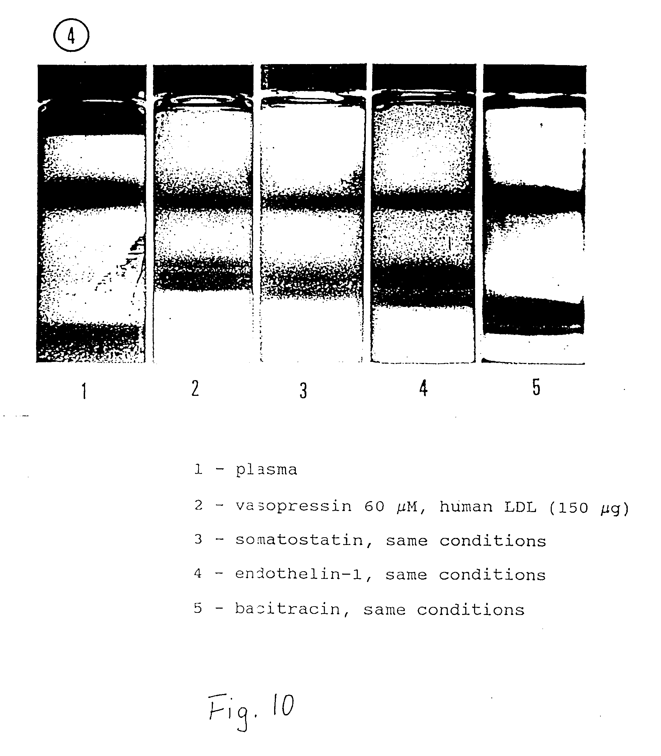

To demonstrate the fusogenic action of *somatostatin* and *endothelin-1*, the same types of assays were employed. *Bacitracin* and *vasopressin* were used as controls (see Figs. 10 and 11). Purified human LDL (0.15 mg/ml) in the same fusion buffer as above was incubated with *bacitracin*, *vasopressin*, *somatostatin* and *endothelin-1* (60 µm final concentrations) at 20°C. In gel filtration, the elution volumes of the fused LDL prepared by all four fusogenic compounds were decreased by approximately 5.5 % indicating an increase in size of the LDL in all cases. Curves of turbidity as a function of time reach plateaux for all four molecules after ten minutes incubation. For *bacitracin* two bands floating at 1.24 and 1.20 g/ml were obtained by KBr gradient ultracentrifugation. For *vasopressin*, *somatostatin* and *endothelin-1*, two bands with flotation densities between 1.17 and 1.20 g/ml were obtained. The density of the bands increased progressively from *vasopressin* to *endothelin*-1. These results collectively indicate that *somatostatin* has a fusogenic capacity comparable to that of *vasopressin*, while *endothelin*-1 has a fusogenic capacity which is more close to that of *bacitracin*. Both *somatostatin* and *endothelin*-1 transform in fusion buffer human LDL particles into heavy microemulsion particles of increased dimensions.

The fusogenic capacity of *bacitracin*, *vasopressin*, *somatostatin* and *endothelin-*1 point to the common structural features necessary for lipoprotein fusion which also have implications for the atherogenic process. On theoretical grounds it can be claimed that the peptides *endothelin-*2 and *endothelin*-3 will have fusogenic properties based on their structure which is similar to *endothelin-*1.

The structural features common to these functionally different molecules indicate that they have a role in the fusogenic activity of the peptides. These features can be summarized as follows:- a globular amino- or carboxy terminus of 6 to 15 amino acids; a structure which inherently forms a cycle or a ring and which is stabilized by one or two disulphide linkages (i.e. this part of the peptide sequence must contain spaced apart cysteine residues) or by residues which have a cycle or ring conformation; and a (positively) charged amino- or carboxy end of 2 to 6 amino acids - an alternative structure of this part of the molecule is a short amphipathic α-helix of 6 to 10 amino acids. The destabilizing properties should also depend on the inverted wedge shape of the globular part of the molecule, a conformation which is characteristic for class L (lytic) peptides (Tyler, E.M., et al, *J. Biol. Chem.*, 1993, 268, 221120). A structural feature in favour of a fusogenic activity should also result from the capability of the last amino acids of the side chain to form a metal chelate with divalent cations. A characteristic example in this connection is the thiazoline ring at the end of the *bacitracin* side chain (Stone, K.J.; Strominger, J.L., *Proc. Natl. Acad. Sci., USA*, 1971, 68, 3223).

*In-vitro fusion by bacitracin*. *Bacitracin* was added to 0.1 - 0.7mg LDL in fusion buffer from a fresh 70 mM solution at a final concentration of 0.35 mM. The preparation was incubated at 20°C for 15 min. Best fusion yield was obtained for a ratio of 7.5 between *bacitracin* and apolipoprotein B. At the end of the incubation period, Ca²⁺ was chelated with ethylene diamine tetraacetic acid (EDTA) (2 Mm final concentration). An extensive dialysis of the preparation against 4,000 volumes of PBS followed. The dialysed preparation was ready for immunization.

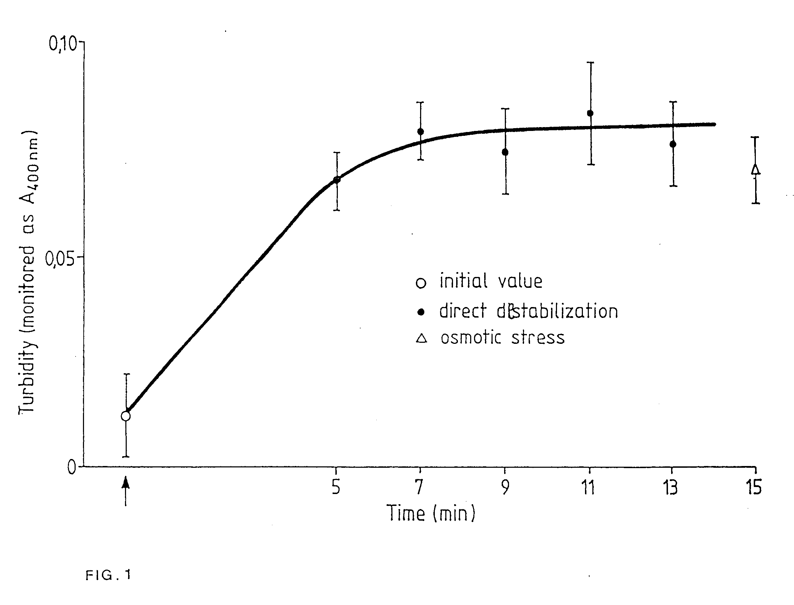

*Bacitracin* produced the LDL fusion in the presence of 1.6 mM Ca²⁺ in 7 min.; the equilibrium was attained for hamster LDL in 15 min and for human LDL in 20 min. This was indicated by the turbidity of the sample, monitored spectrophotometrically by absorption at 400 nm (Figure 1). *Bacitracin* was effective in inducing the LDL fusion from 0.35 mM to 0.35 pM.

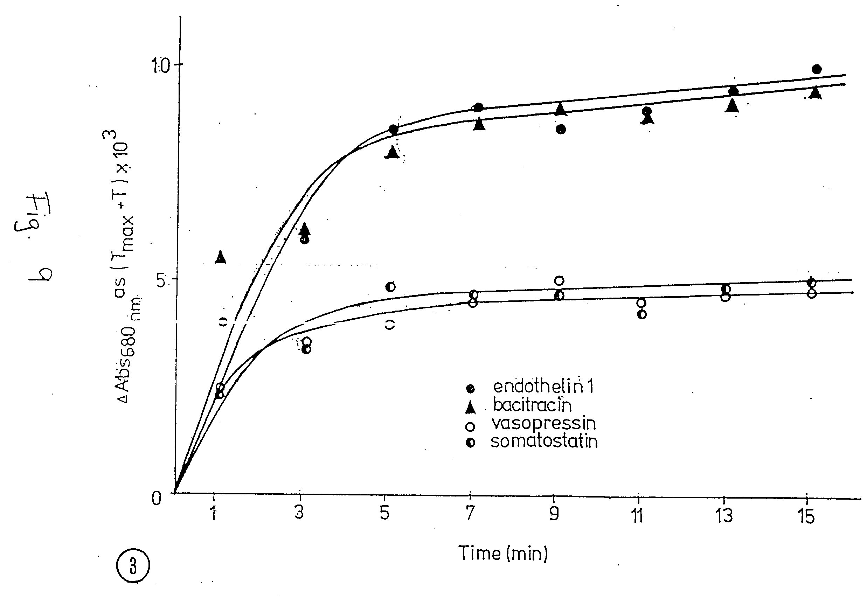

Heavy microemulsion particles were also obtained by the fusion of human VLDL with *bacitracin*. These particles were obtained at 20°C from native purified human VLDL (0.2 mg/ml), in a phosphate fusion buffer containing 72 mM Na⁺, 2 mM K⁺, 0.3 mM Mg⁺ and 1.6 mM Ca²⁺, after incubation with 0.88 mM *bacitracin* (final concentration) for 17 min. The formation of the heavy microemulsion particles was tested by gel filtration, turbidimetry and ultracentrifugation on a KBr density gradient. Gel filtration analysis was performed on a 10 cm x 1.5 cm diameter Sephadex G-150 column, calibrated for the elution volumes with purified human lipoproteins (VLDL, LDL, IDL). The fusion of the VLDL decreased the elution volume of VLDL by 6% indicating an increase in particle dimensions. Turbidimetry was monitored as the difference in absorption at 680 mn; the turbidity reached a plateau in 7 minutes. Ultracentrifugation analysis showed that a 1.24 g/ml band was obtained after VLDL fusion, indicating the formation of heavy microemulsion particles (see Fig. 9).

Heavy microemulsion particles were further obtained by fusion, under the same condition as above, of a mixture of isolated purified VLDL (0.1 mg/ml) and LDL (0.1 mg/ml). The fusion was monitored by following the turbidity which reached a plateau in 17 minutes. Ultracentrifugation analysis showed that a 1.24 g/ml band was obtained indicating the formation of heavy heterogenous microemulsion particles.

*Fusion by vasopressin*. *Vasopressin* (Sigma Chemical Co., USA) was added to 0.1 - 0.7 mg/ml LDL in fusion buffer FB from a fresh 1 mg/ml solution to a final concentration of 2 pg/ml; the preparation was incubated at 20°C for 30 min. An extensive dialysis against 4,000 volume of PBS followed. The concentration of *vasopressin* tested was in the physiological range (2 pg/ml).

### In vitro fusion by osmotic stress

The second method of LDL or lipid fusion is based on an osmotic stress inducing the fusion process by osmoelastic coupling. The osmotic stress was generated by subjecting the LDL, transferred in FB to a dialysis against 20% polymer solutions of polyethylene glycol (PEG) 20,000 or dextran 40,000.

The 0.1 - 0.7 mg/ml LDL solution in FB was subjected to a 15 min dialysis at 20°C against a 20% solution of PEG 20,000 or dextran 40,000 in water. The cutoff limit of the dialysis bag was 10,000. An extensive dialysis against 4,000 volumes of PBS followed.

### Results

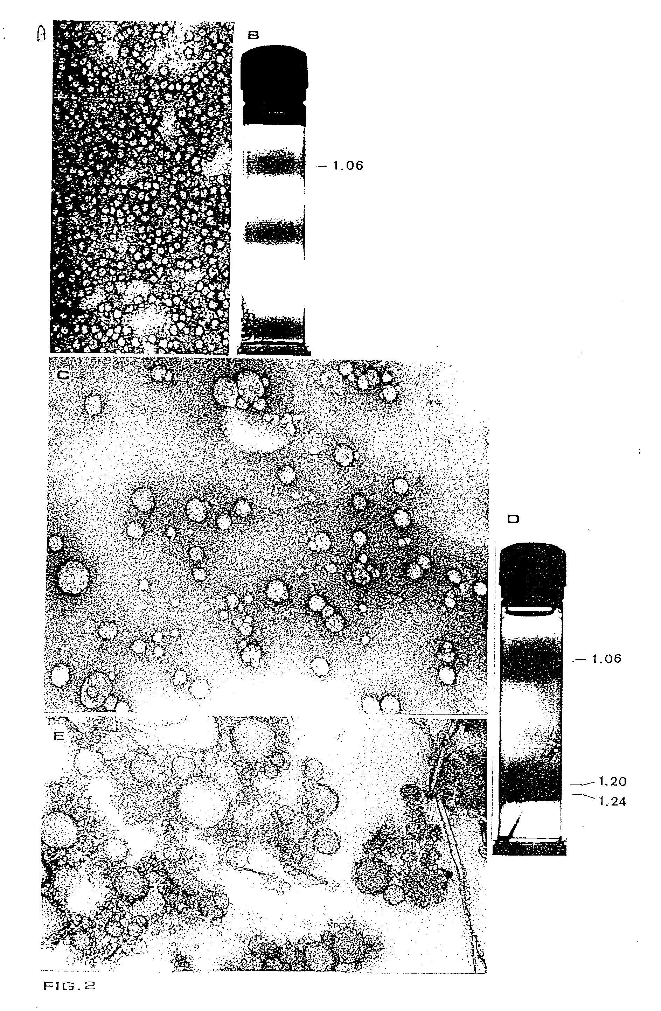

The fusion phenomena were observed ultrastructurally by negative staining. The LDL preparation was formed from 20 nm particles of uniform size (Figures 2A), floating at 1.06 g/ml in the gradient density ultracentrifugation (Figures 2B). The fused preparation was formed from microemulsion droplets with dimensions ranging from 30 to 300 nm (Figure 2C), floating in two bands at 1.21 and 1.24 g/ml (Figure 2D). The dimensions and the flotation densities of the microemulsion droplets in the fused preparations were similar to the material isolated from the experimental hamster atherogenic lesions. It was also concluded that the change in the flotation density in both *in vivo* and *in vitro* material was due to the presence of multiple copies of apolipoprotein B on the surface of the microemulsion droplets. This conclusion was based on a correlative investigation of both *in vivo* and *in vitro* material from the experimental animals and of *in-vitro* studies for the human material; this correlative investigation comprised biochemistry, cytochemistry, immunochemistry, electron microscopy and work *in vitro* with isolated cells (hamster monocytes).

The heavy microemulsion particles proved to have the same pattern of organization as the LDL; a core of neutral lipid stabilized in a hydrophilic medium by a phospholipid monolayer (Figure 2E) in which multiple copies of the apolipoprotein B were embedded. By taking the LDL parameters as a basis, an estimation of the droplet surface/LDL surface would predict 2 copies apolipoprotein B on the surface of a 30 nm particle and 225 copies on the surface of a 300nm particle. This novel rearrangement of the molecules proved to be antigenic and immunogenic.

### Immunization

Animals were immunized by a conventional immunization scheme comprising a primary immunization with 40µg of protein/animal (mean weight 100 g) and three secondary immunizations at two-week intervals with 25µg of protein each with the microemulsion obtained *in vitro*; a group was hyperimmunized and received 7 secondary immunizations. Then, these animals were given a hypercholesterolemic diet in the form of standard hamster chow supplemented with 5% cholesterol and 15% butter for 7, 14, 30 and 60 days. After these diets, 2-3 ml of blood were obtained by cardiac puncture for biochemical and immunochemical analysis. The animals were then sacrificed for ultrastructural investigation of the lesions.

The total cholesterol determinations in the sera of these animals indicated clearly their hypercholesterolemic state, by comparison with the values obtained from atherogenic control animals. The only significant deviation from the control values was at 7 and 14 days of diet, when a significant increase in the level of circulating cholesterol was observed. The increase in the level of circulating cholesterol can be explained by an immunosuppression phenomenon due to the unused LDL present in the preparation. The immunosuppression was not present in animals immunized with fused human LDL (Table 1).

**TABLE 1**

| | Serum Cholesterol Values (mg/dl) in Atherogenic Animals | | | |
|---|---|---|---|---|
| Time on Hypercholestolemic Diet (days) | Control (saline) | Standard Immunisation Regimen with Fused LDL | Hyperimmunised Regimen with fused LDL | Standard Immunisation Regimen with Fused Human LDL |
| 0 | 103 ± 7 | " | " | " |
| 7 | 150 ± ? | 327 ± 72 | " | 126 ± 38 |
| 14 | 200 ± ? | 419 ± 73 | " | 146 ± 16 |
| 30 | 250 ± ? | 258 ± 49 | 240 ± 76 | 244 ± 13 |
| 60 | 300 ± ? | 352 ± 96 | " | 244 ± 13 |

After the initial increase the cholesterol level dropped and reached values comparable to that of the atherogenic unimmunized control.

The circulating anti-microemulsion antibody titers, determined by conventional enzyme-linked immunosorbent assay (ELISA), showed measurable but very low antibody titers (2 to -3) at 7 days of diet. At all other diet times, no measurable levels of circulating antibodies were detected, which indicates that the immune reaction was confined to the lesional areas, as will be detailed below. The cross-reactivity of the sera from the antigen-challenged animals (immunized animals receiving an atherogenic diet) with the homologous LDL was tested and a negative result was obtained.

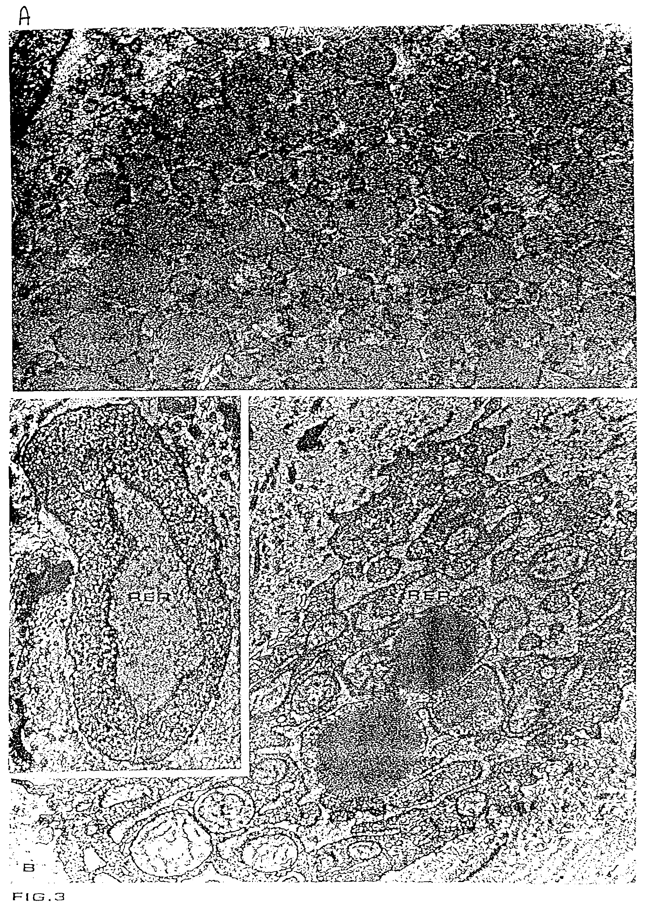

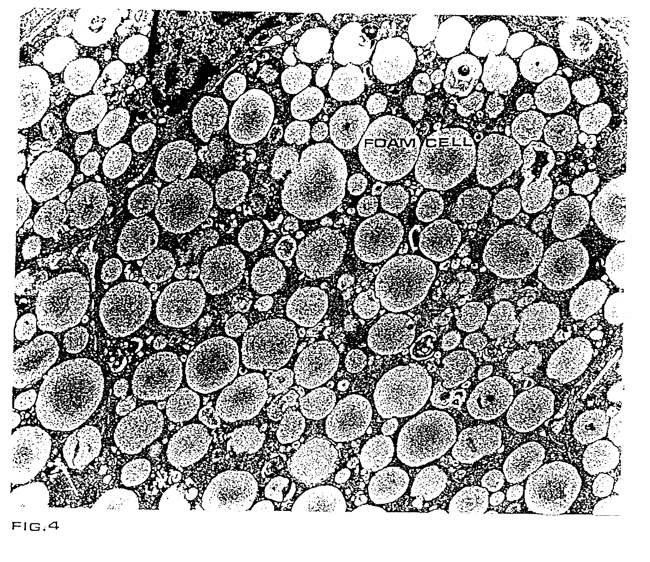

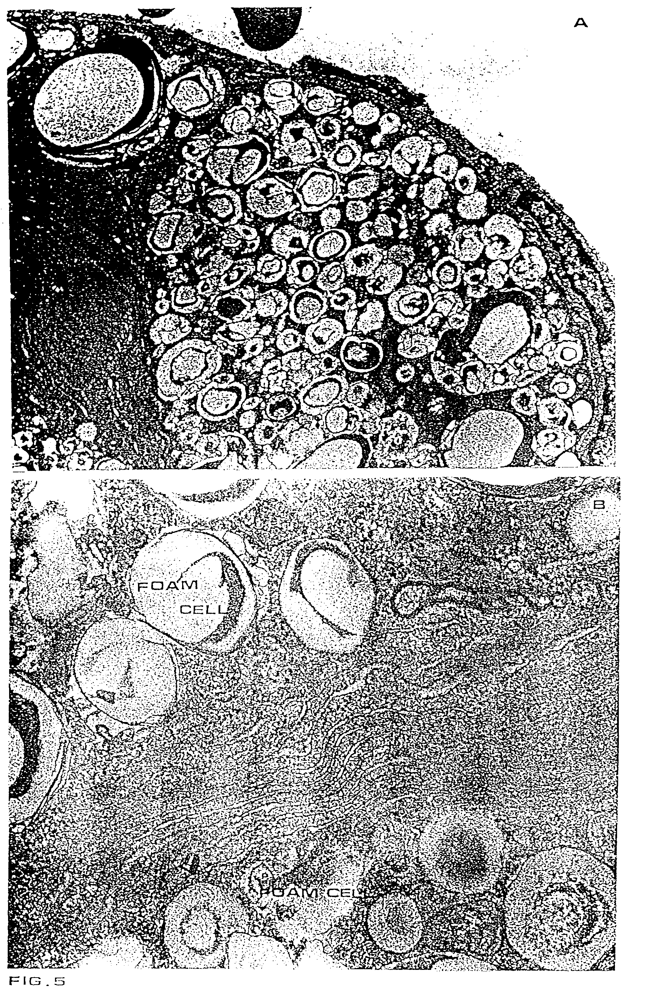

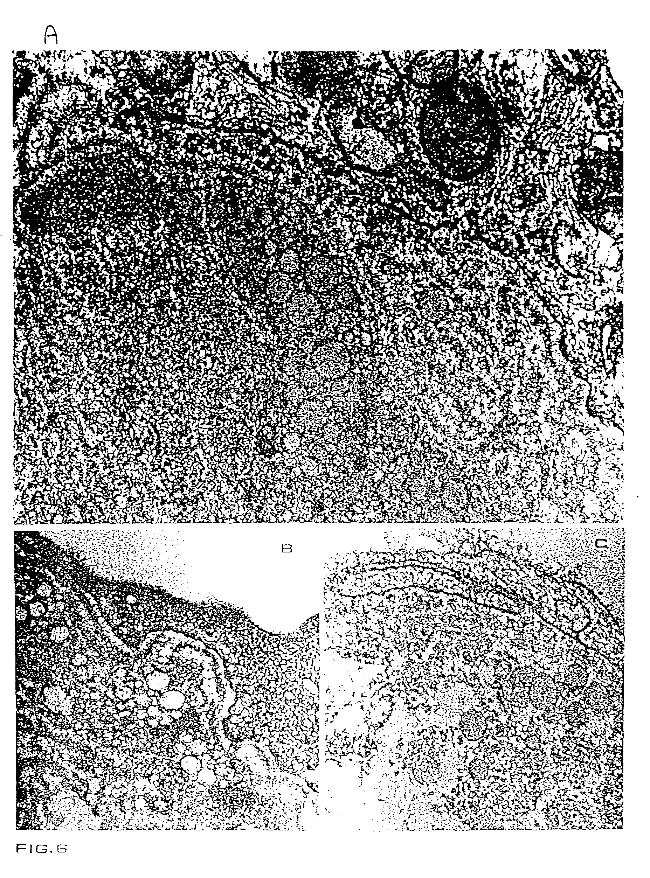

The effect of the immunization was tested on lesion development in the region of the aortic valve rings. This area is particularly susceptible to the atherogenic diet, developing fibrolipic-like lesions 10 times faster than any other lesion-prone areas in the hamster. Thus, experimental animals were fed an atherosclerotic diet whereupon they received a subcutaneous injection at two-weekly intervals. The animals were maintained on the atherogenic diet until sacrifice. The main body of these lesions was formed by the extracellular lipid material in a very dynamic state of lipid component rearrangement. In the antigen-challenged (immunised) animals at 7 days of diet, the lesions developed in the above locations, implying that the LDL metabolism in these animals was not affected, being similar to the control atherogenic animals. At this time of diet, in the controls, the extracellular lipid material was in the form of densely packed microemulsion droplets in a dynamic state of reorganization. In the antigen-challenged animals the microemulsion lipid material was stabilized (Figure 3A) and numerous plasma cells surrounded the lipid deposits (Figure 3B). They had characteristic distensions of their endoplasmic reticulum, indicating an active antibody synthesis (Figure 3B, inset). The reaction of the immune system to the microemulsion droplet material in the lesions in the antigen-challenged animals implied an identity between the microemulsion droplet material formed *in vivo* in the lesions and the *in-vitro* material used for the immunizations, since the diet was administered post-immunization. At 14 days of diet, the former fibrolipidic-like lesions developed in the unimmunized controls were replaced by a fatty streak (Figure 4), implying that a reversion of the lesion type took place. This was not previously induced in any experimental model of atherogenesis by any type of experimental manipulation and is a process only presumed to take place naturally in humans in which, occasionally, lesional areas requilibrate (Munro, J.M. Cotran, R.S., *Lab. Invet.*, 1988, 58, 249). At 30 and 60 days of diet, 80% of the aortic ring area looked like normal. Moreover, the clearance of the area was assured by numerous monocyte-derived cells, showing morphological specializations which clearly indicated a close cooperation in their regression from the tissue (Figure 5A and 5B).

In animals immunized with the fused human LDL the same trend of events was observed, and the presence of antibody secreting B cells was the same around the lesions. Consequently the same reversion events took place and at about 1 month of diet the vessel returned to its normal structure.

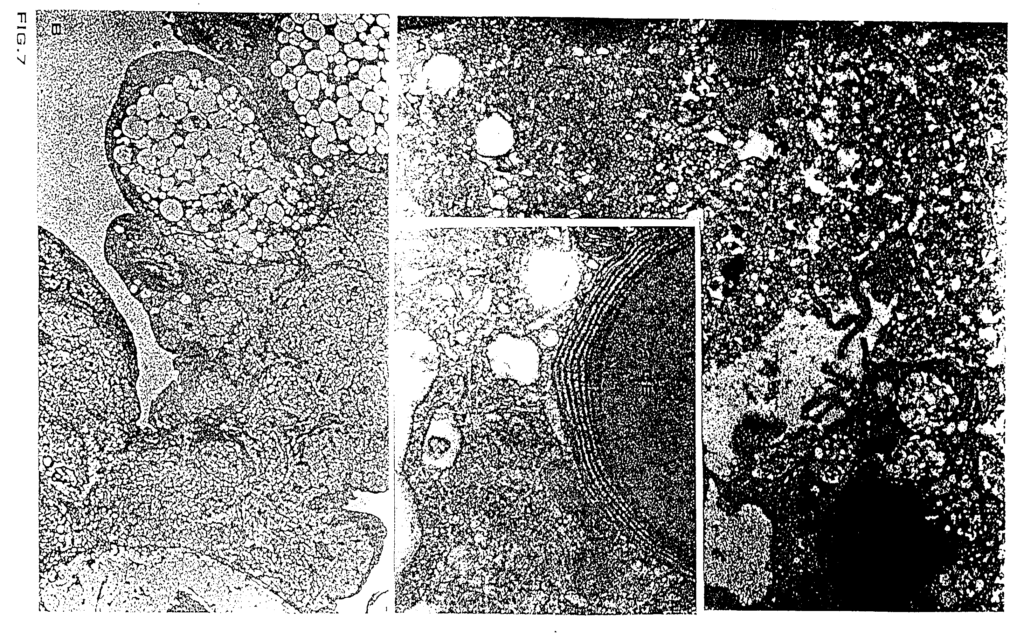

In the hyperimmunized animals, at 30 days of antigen-challenge post-immunization, the structure of the aortic ring areas was normal, as compared to the normal animals (not immunized and immunized controls) and with the immunized controls (immunized animals receiving a normal diet) (Figures 6A, 6B, 6C).

In a special group of animals, the immunization was applied post-diet to elucidate if the same reversion process could be induced. After 3 months of atherogenic diet these animals had a total plasma cholesterol of 281 ± 95 mg/dl. By taking into consideration the moment of activation of the resident valvular macrophages into presenting the antigen, a process which appears from the first week of diet in the antigen-challenged animals and appears very late (6 months) in the course of atherogenic lesion development (Flip, D. A., et al. *Atherosclerosis*, 1987 67, 199).

Our working hypothesis indicated that a reasonable moment to check the eventual reversion was at 1/10 of the total diet time from the moment the immunization was completed (the immune surveillance system established). A considerable degree of reversal was obtained in comparison with the atherogenic controls (Figure 7A). However, the previous diet period left sequelae, representing the effects of lipid component reorganization (Figure 7B).

Nevertheless, even the reorganized lipid material in the form of microscobic cholesterol ester droplets (Figure 7B) was taken up by the monocyte/macrophages in the same time with the phagocytosis of the heavy microemulsion formed *in vivo* by the fusion. The mechanism of disposal of cholesterol ester microbic droplets was similar to the one observed *in vitro* during the incubation of monocyte/macrophages with cholesterol crystals (Koren, E. et al, *Prgo. Lipid Res.*, 1991 30, 1237). It consisted in the surrounding of the cholesterly ester microbic droplets with multiple phospholipid bilayers (Figure 7B, insert).

In conclusion, it appears that the immunization against a heavy microemulsion formed by LDL fusion, representing *in vivo* the initial step for the accumulation of extracelluar atherogenic lipids in lesion-prone areas, reversed the evolution of atherosclerosis simply by blocking the initial step. This immunological approach proved beneficial for the management of atherogenic lesions even if applied in advance of post-diet. Therefore, this approach may represent a prophylactic or therapeutic treatment of atherogenesis by a vaccination type mechanism.

## Claims

1. A pharmaceutical composition comprising heavy microemulsion lipid particles, obtainable by
(i) *isolation* of low density lipoproteins (LDL) and/or very low density lipoproteins (VLDL) and/or intermediate density lipoproteins (IDL), all preferably of human origin; and
(ii) *in vitro* fusion of said lipoprotein material to obtain heavy microemulsion lipid particles, having a floatation density of 1.17 to 1.24 mg/ml.

2. A pharmaceutical composition according to claim 1 wherein the LDL, VLDL or IDL fusion step is obtained by a surface active compound with amphipathic properties and wherein said material is added to fuse the lipoproteins and then removed.

3. A pharmaceutical composition according to claim 1 or claim 2, wherein the amphipathic material is selected from *bacitracin, vasopressin, somatostatin* and *endothelins*.

4. A pharmaceutical composition according to claim 3 wherein said amphipathic material is removed by dialysis.

5. A pharmaceutical composition according to claim 1, wherein the *in vitro* fusion step is obtained by osmotic stress inducing the fusion process by osmoelastic coupling.

6. A pharmaceutical composition according to any preceding claims wherein the heavy microemulsion particles have dimensions between 30 and 300 nm.

7. A pharmaceutical composition for treatment and prophylaxis of atherosclerosis comprising as active ingredient a reorganized lipid material originating from potentially atherogenic lipid material of the mammal or human subject to be treated and an immunization adjuvant.

8. A pharmaceutical composition as claimed in any one of the preceding claims, having the galenic form of an injection.

9. A method for preparing a pharmaceutical composition for treatment and prophylaxis of atherosclerosis according to any one of the preceding claims, wherein the active ingredient is obtained by adding *in vitro* amphipathic molecules to an isolation of potentially atherogenic lipoprotein material to obtain a reorganization of the lipid material.

10. A method for preparing a pharmaceutical composition for treatment and prophylaxis of atherosclerosis according to any one of the preceding claims, wherein the active ingredient is obtained by inducing osmotic stress in an isolation of potentially atherogenic lipoprotein material to obtain a reorganization of the lipid material.
